# FASCICULE DE BREVET EUROPEEN

(11) **EP 1 180 134 B1**
(45) Date de publication et mention de la délivrance du brevet: **08.09.2004**
(21) Numéro de dépôt: 00927346.7
(22) Date de dépôt: 16.05.2000
(51) Int. Cl.: C12M 1/36, C12N 5/06, C12N 5/08

(54) **PROCEDE ET DISPOSITIF DE CULTURE DE CELLULES A APPLICATIONS MULTIPLES**
ZELLKULTURVORRICHTUNG UND -VERFAHREN MIT MEHRFACHANWENDUNGEN
MULTI- APPLICABLE CELL-CULTURE METHOD AND APPARATUS

(30) Priorité: 26.05.1999 FR 9906820
(43) Date de publication de la demande: 20.02.2002
(73) Titulaire: Bertin Technologies, 75008 Paris (FR); ASSISTANCE PUBLIQUE, HOPITAUX DE PARIS, 75004 Paris (FR)
(72) Inventeur: DOUAY, Luc, F-94100 Saint-Maur (FR); MAGALHAES, Jean-Luc, F-78490 Boissy sans Avoir (FR); HUMMEL, Florence, F-78360 Montesson (FR)
(74) Mandataire: Ramey, Daniel
(86) Numéro de dépôt international: PCT/FR2000/001311
(87) Numéro de publication internationale: WO 2000/073411

(56) Documents cités:
- WO-A-91/15570
- WO-A-92/13940
- WO-A-96/40858
- WO-A-99/28438

## Description

L'invention concerne un procédé et un dispositif de culture de cellules à applications multiples, destinés notamment mais non exclusivement à la culture de cellules hématopoïétiques (cellules sanguines, progéniteurs hématopoïétiques et cellules souches), au moyen d'un bioréacteur, tel par exemple que celui décrit dans le document EP-A-0474847.

Les procédés et dispositifs de ce type ont été beaucoup étudiés dans la technique antérieure, en raison de l'intérêt qu'ils présentent pour diverses applications telles que les greffes, la production de types cellulaires particuliers, etc...

On connaît notamment, par la demande internationale WO96/40858 de AASTROM BIOSCIENCES INC., un dispositif de culture de cellules biologiques, en particulier de cellules hématopoïétiques, comprenant une cassette dans laquelle est formée une chambre de culture, un réservoir d'un milieu nutritif contenant des facteurs de croissance, un réservoir destiné à recevoir le milieu usé, un sac de récolte de cellules et des moyens de connexion entre ces composants pour former un système fermé stérile. Ce dispositif est d'abord utilisé sur un premier appareil pour le remplissage de la chambre de culture par le milieu nutritif, l'inoculation de cellules dans la chambre de culture, la répartition des cellules dans la chambre par agitation, puis sur un deuxième appareil (un incubateur) pendant deux semaines au cours desquelles la chambre de culture et le réservoir de milieu nutritif sont maintenus à des températures prédéterminées, le milieu nutritif, de l'oxygène et d'autres gaz sont amenés dans le dispositif avec des débits prédéterminés, les paramètres de température et de débit étant définis pour correspondre à des conditions optimales de croissance des cellules et étant pour certains d'entre eux susceptibles de varier dans le temps conformément à un programme pré-établi.

L'inconvénient essentiel de ce dispositif connu est son manque d'adaptabilité et de variabilité, qui a pour conséquence que ce dispositif est "figé" et destiné à une application spécifique, notamment en raison du fait que :
- le milieu nutritif utilisé est un mélange prédéfini et non modifiable en cours d'application, contenant des facteurs de croissance qui sont dilués et répartis de façon homogène dans le mélange de sorte que, ni la composition du milieu nutritif, ni la quantité de facteurs de croissance qu'il contient ne peuvent être modifiées après le début de l'application,
- il est impossible de régler séparément les débits de milieu nutritif et de facteurs de croissance amenés dans la chambre de culture,
- le programme qui définit une variation éventuelle du débit de milieu nutritif est pré-établi (il est enregistré dans une mémoire fournie avec le dispositif), et ne peut être modifié en cours d'opération ou avant le lancement d'une opération,
- la composition et le débit du mélange de gaz amené dans le dispositif sont prédéfinis et invariables en cours d'application,
- le dispositif est réalisé sous la forme d'un "kit" à usage unique et automatisé, destiné à une application spécifique et utilisable par du personnel médical non spécialisé à qui il est demandé de suivre à la lettre un mode d'emploi sans prendre la moindre initiative,
- aucun contrôle du processus de culture cellulaire n'est possible en cours d'application.

La présente invention a des objectifs tout à fait différents.

Elle a pour objet un procédé et un dispositif de culture de cellules permettant de modifier à volonté et à tout moment non seulement le débit, mais aussi la composition ou la nature d'un milieu nutritif amené dans une chambre de culture.

Elle a également pour objet un procédé et un dispositif du type précité, qui permettent de plus de modifier à volonté et à tout moment la composition et le débit d'un mélange de facteurs de croissance amené dans la chambre de culture, ces modifications étant indépendantes des modifications précitées de la composition et du débit de milieu nutritif.

Elle a encore pour objet un procédé et un dispositif du type précité, qui permettent de modifier à volonté et à tout moment la composition et le débit du mélange de gaz de ventilation amené dans la chambre de culture, ces modifications étant indépendantes des modifications précitées de la composition et du débit de milieu nutritif et de la composition et du débit du mélange de facteurs de croissance.

Elle a encore pour objet un procédé et un dispositif du type précité, qui sont conçus et réalisés pour avoir une pluralité d'applications différentes et non pour une application spécifique et unique.

Elle a encore pour objet un procédé et un dispositif du type précité, qui sont utilisables aussi bien comme outil de recherche que pour une application clinique ou autre, comme par exemple en milieu hospitalier pour la culture de cellules prélevées sur un patient ou un donneur, en vue d'une réinjection ultérieure.

Elle a encore pour objet un procédé et un dispositif du type précité, qui sont utilisables, moyennant des modifications mineures, avec des chambres de culture de volumes différents, couvrant une gamme allant de 1 millilitre à 1 litre environ.

Elle propose, à cet effet, un procédé de culture de cellules à applications multiples, en particulier pour l'entretien, la prolifération, l'amplification ou la différenciation de cellules en enceinte fermée, consistant à introduire des cellules dans cette enceinte, à l'alimenter par des débits de gaz de ventilation, de milieu nutritif et de facteurs de croissance, et à récolter les cellules cultivées, caractérisé en ce qu'il consiste également :
- à disposer de plusieurs sources de composants ou de milieux nutritifs différents et de plusieurs sources de facteurs de croissance différents,
- à déterminer des compositions et des débits de mélanges des différents composants ou milieux nutritifs et des compositions et des débits de mélanges des différents facteurs de croissance, correspondant à l'une envisagée des applications précitées,
- puis, au cours du déroulement de ladite application, à modifier à intervalles réguliers ou non les compositions et/ou les débits des mélanges de composants ou de milieux nutritifs et des mélanges de facteurs de croissance amenés dans l'enceinte, ces modifications pouvant être effectuées indépendamment les unes des autres.

De façon surprenante, on a constaté que la quantité de facteurs de croissance introduite dans un bioréacteur de culture de cellules est un paramètre sensible et critique au niveau de l'expansion des cellules et que des variations même relativement faibles de ce paramètre autour d'une valeur optimale ont des effets notables sur l'expansion des cellules et la réduisent de façon importante, alors que ces mêmes variations n'ont pratiquement pas d'influence sur l'expansion des cellules dans un système de culture statique (culture en poches). Il est donc particulièrement important, dans un système de culture à perfusion continue ou discontinue tel qu'un bioréacteur, de pouvoir régler avec précision les quantités de facteurs de croissance amenées dans le bioréacteur. L'invention permet ces réglages pendant toute la durée d'une culture, quel que soit le type d'application prévue (entretien, prolifération, amplification ou différenciation de cellules).

Il en est de même en ce qui concerne la composition et le débit du milieu nutritif avec lequel on alimente le bioréacteur, l'invention permettant de faire varier et de régler à volonté cette composition et ce débit.

En particulier, cette alimentation en milieu nutritif peut être continue ou discontinue.

Il en résulte d'une part une augmentation sensible du rendement d'un bioréacteur et d'autre part la possibilité d'utiliser un même type de bioréacteur pour différentes applications.

Selon une autre caractéristique de l'invention, le procédé consiste également à disposer de plusieurs sources de gaz différents, à déterminer une composition et un débit d'un mélange des différents gaz correspondant à l'application envisagée, puis à alimenter l'enceinte par ce mélange de gaz et, à intervalles réguliers ou non au cours du déroulement de l'application, à modifier si nécessaire la composition et/ou le débit du mélange de gaz amené dans l'enceinte.

Ces possibilités de modification du mélange de gaz alimentant l'enceinte sont favorables à l'augmentation du rendement d'un bioréacteur et à la diversité des applications envisageables.

Selon encore une autre caractéristique de l'invention, ce procédé consiste également à contrôler, de façon périodique ou non, le développement des cellules dans ladite enceinte au cours du déroulement de l'application précitée et à modifier les compositions et/ou les débits des mélanges de milieux nutritifs, de facteurs de croissance et de gaz en fonction des résultats de ces contrôles.

Ces caractéristiques du procédé selon l'invention permettent de définir pour chaque application, des conditions optimales d'alimentation d'une chambre de culture de cellules et des variations optimales de ces conditions dans le temps, de contrôler à intervalles périodiques ou non le déroulement de l'application et, en fonction des résultats de ces contrôles, de modifier autant que nécessaire et aussi souvent que nécessaire ces conditions d'alimentation et leurs variations.

Il en résulte une nette amélioration du rendement des cultures de cellules dans les diverses applications possibles.

L'invention propose également un dispositif de culture de cellules à applications multiples, en particulier pour l'entretien, la prolifération, l'amplification ou la différenciation de cellules, comprenant une enceinte fermée, des membranes microporeuses délimitant une chambre de culture dans ladite enceinte, des moyens d'introduction de cellules dans ladite chambre, des moyens d'alimentation de l'enceinte en gaz de ventilation, en milieu nutritif et en facteurs de croissance, et des moyens de collecte des cellules, caractérisé en ce qu'il comprend également des réservoirs de milieux ou de composants nutritifs différents, reliés par des moyens de réglage de débit à un collecteur d'alimentation de ladite enceinte, des réservoirs de facteurs de croissance différents, reliés par des moyens de réglage de débit à un autre ou au même collecteur d'alimentation de l'enceinte, et des moyens de commande des moyens de réglage précités pour déterminer, en fonction d'une application envisagée, la composition et/ou le débit d'un mélange de milieux ou de composants nutritifs et la composition et/ou le débit d'un mélange de facteurs de croissance amenés dans l'enceinte par le ou les collecteurs d'alimentation précités et pour modifier ces compositions et/ou ces débits au cours du déroulement de l'application.

Avantageusement, ce dispositif comprend également des réservoirs de gaz différents, des moyens individuels de réglage de débit reliant ces réservoirs à un collecteur d'alimentation de ladite enceinte et des moyens de commande des moyens de réglage pour déterminer la composition et/ou le débit d'un mélange de gaz amené dans ladite enceinte en fonction de l'application envisagée et pour modifier si nécessaire cette composition et/ou ce débit de gaz au cours du déroulement de l'application.

L'invention sera mieux comprise et d'autres caractéristiques, détails et avantages de celle-ci apparaîtront plus clairement à la lecture de la description qui suit, faite à titre d'exemple en référence aux dessins annexés dans lesquels :
- la figure 1 est une vue schématique d'un dispositif de culture de cellules selon l'invention ;
- les figures 2a, 2b et 2c sont des graphes illustrant l'influence, dans une culture en bioréacteur, de la quantité de facteurs de croissance sur l'expansion de cellules nucléés, de progéniteurs granulomacrophagiques et de cellules souches ;
les figures 3a, 3b et 3c illustrent cette même influence dans une culture statique en poches.

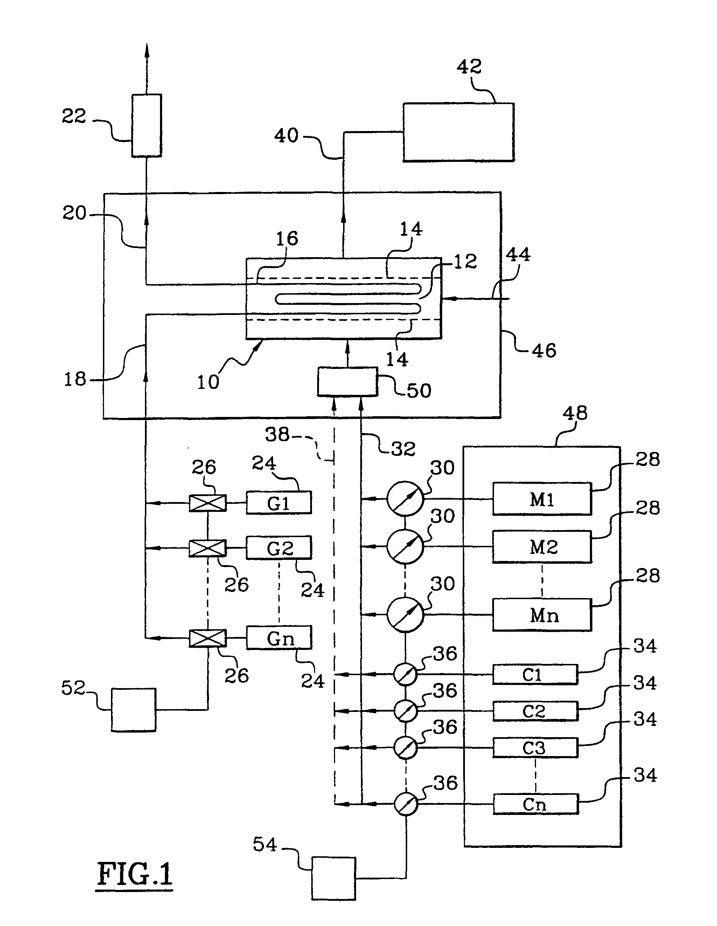

Le dispositif de culture représenté schématiquement en figure 1 est destiné en particulier, mais non exclusivement, à la culture, la prolifération, la différenciation et/ou l'amplification de cellules biologiques, en particulier de cellules hématopoïétiques, ce dispositif comprenant essentiellement un bioréacteur qui est de préférence du type décrit dans le document EP-A-0474847 ou dans le document WO99/28438.

Pour l'essentiel, ce bioréacteur (dont on trouvera une description détaillée dans les documents précités) comprend une enceinte fermée 10 à usage unique dans laquelle une chambre de culture 12 est délimitée par des membranes microporeuses 14 ayant une taille de pores comprise entre 0,1 et 5 µm environ, formant des barrières de confinement des cellules hématopoïétiques et dont les caractéristiques sont déterminées en fonction du type de cellules à cultiver.

Au moins une nappe de tubes capillaires 16 de circulation de gaz est disposée dans la chambre de culture 12, ces tubes étant des fibres creuses poreuses qui sont réparties régulièrement entre les membranes 14 et qui sont reliées d'une part, en entrée, à un collecteur d'alimentation 18 relié lui-même à des moyens d'alimentation en gaz, et d'autre part, en sortie, à un collecteur de sortie 20 raccordé à un réservoir 22 de stockage temporaire de gaz sortant, que l'on peut éventuellement analyser pour contrôler le déroulement d'une culture de cellules dans la chambre 12.

Les moyens d'alimentation en gaz comprennent, selon l'invention, plusieurs réservoirs 24 contenant sous pression des gaz différents G1, G2, ... , Gn (par exemple de l'oxygène, de l'azote, du CO₂, ...) et dont chacun est relié par un moyen individuel 26 de réglage de débit au collecteur d'alimentation 18.

L'enceinte 10 est d'autre part équipée de moyens d'alimentation en milieu nutritif et en facteurs de croissance et de moyens de sortie de milieu nutritif usé.

Les moyens d'alimentation en milieu nutritif et en facteurs de croissance comprennent plusieurs réservoirs ou récipients 28 contenant des milieux nutritifs différents M1, M2, ... Mn (ou des composants différents de milieu nutritif) et qui sont chacun reliés par des moyens individuels 30 de réglage de débit ou de quantité à un collecteur 32 d'alimentation qui forme mélangeur et qui débouche dans l'enceinte 10 en-dessous de la chambre de culture 12, dans l'exemple représenté. Ces moyens d'alimentation comprennent également des récipients 34 contenant des facteurs de croissance différents, par exemple des cytokines différentes C1, C2, C3, ..., Cn, chaque récipient 34 étant relié par un moyen individuel 36 de réglage de quantité ou de débit à un collecteur d'alimentation qui peut être le collecteur 32 utilisé pour l'alimentation en milieux nutritifs ou un collecteur différent, tel que celui représenté en 38 en trait pointillé, ce collecteur 38 débouchant dans l'enceinte 10 en-dessous de la chambre de culture 12, comme le collecteur 32, ou bien étant relié à celui-ci en amont de l'enceinte 10 éventuellement au moyen d'une chambre de mélange.

Les quantités utilisées de facteurs de croissance sont très faibles, ce qui amène à diluer ces facteurs de croissance dans des milieux appropriés, comme par exemple le milieu nutritif précité, pour pouvoir régler plus facilement et plus précisément les quantités ou les débits à introduire dans l'enceinte 10.

Un collecteur 40 de sortie de milieu usé relie la partie supérieure de l'enceinte 10, au-dessus de la chambre de culture 12, à un réservoir ou récipient 42.

Dans certains cas, une partie du milieu usé sortant de l'enceinte 10 par le collecteur 40 peut être filtrée et réinjectée dans cette enceinte, en-dessous de la chambre de culture 12.

Une paroi latérale de l'enceinte 10 est équipée de moyens 44 (schématisés ici par une flèche) d'injection ou d'inoculation de cellules à l'intérieur de la chambre de culture 12 et de prélèvement ou de récolte des cellules cultivées dans la chambre 12, ces moyens 44 étant d'un type connu permettant d'injecter des cellules ou d'en prélever dans la chambre de culture 12 de façon stérile.

L'enceinte 10 est placée à l'intérieur d'une enceinte 46 thermostatée, qui est réglée à la température de culture souhaitée et à l'extérieur de laquelle se trouvent les moyens d'alimentation en gaz, en milieu nutritif et en facteurs de croissance et les réservoirs 22 de gaz sortant et 42 de milieu usé. Les récipients 28 contenant les milieux nutritifs ou leurs composants et 34 contenant les facteurs de croissance sont eux-mêmes placés à l'intérieur d'une enceinte réfrigérée 48.

A l'extérieur de l'enceinte thermostatée 46, le ou les collecteurs d'alimentation débouchent dans un réservoir tampon 50 qui forme chambre de dégazage et de remise en température et qui est relié à l'alimentation de l'enceinte 10, ce qui permet d'introduire dans la chambre de culture un milieu nutritif et des facteurs de croissance ayant la température souhaitée pour la culture (par exemple 37° C).

Les moyens 26 précités de réglage des débits des différents gaz G1, G2, ..., Gn sont des vannes commandées d'un type connu, permettant de régler la pression et le débit de chaque gaz dans le collecteur d'alimentation 18.

Les moyens 30 et 36 de réglage des débits ou des quantités de milieux nutritifs et de facteurs de croissance introduits dans le ou les collecteurs d'alimentation 32, 38 sont des micro-pompes commandées telles que des pompes péristaltiques qui permettent de conserver la stérilité des milieux nutritifs et qui peuvent fonctionner sur des gammes de débit étendues, le débit de milieux nutritifs pouvant varier de 1 à 4 fois environ le volume de la chambre de culture par jour, ce qui correspond, pour une chambre de culture de 3 ml à des débits d'environ 0,1 à 0,5 ml/h par exemple, les alimentations en milieux nutritifs et en facteurs de croissance pouvant être continues ou discontinues. On peut également utiliser pour ces alimentations des moyens d'injection commandés, du type pousse-seringue.

Des moyens de commande 52, 54, avantageusement pilotés par ordinateur, sont associés aux divers moyens 26, 30 , 36 de réglage de débit ou de quantité décrits dans ce qui précède pour, d'une part, prédéfinir des conditions optimales d'alimentation de l'enceinte 10 en gaz de ventilation, en milieu nutritif et en facteurs de croissance ainsi que des variations optimales dans le temps de ces conditions d'alimentation et pour, d'autre part, modifier ces conditions d'alimentation et leurs variations en fonction des résultats de contrôles éventuels effectués à intervalles périodiques ou non, du déroulement d'une culture à l'intérieur de la chambre 12. Ces contrôles peuvent être effectués soit en utilisant les moyens 44 de prélèvement de cellules dans la chambre de culture 12, soit sans intrusion dans la chambre de culture 12 en utilisant des moyens optiques d'un type connu.

La multiplicité des possibilités de réglage des compositions et des quantités ou des débits de gaz, de milieu nutritif et de facteurs de croissance introduits dans l'enceinte 10 permet d'adapter le dispositif selon l'invention à des applications diverses, telles par exemple que l'entretien de cellules (consistant à maintenir vivantes des cellules pendant un certain temps in vitro), la prolifération (phase de multiplication d'une cellule), l'amplification (augmentation du nombre de cellules en conséquence de la prolifération), et la différenciation (obtention de cellules matures ayant une spécificité de type et de fonction).

On peut par exemple alimenter la chambre de culture par un mélange d'un milieu nutritif et de plusieurs cytokines, faire varier le débit d'alimentation conformément à un programme pré-établi et/ou en fonction de contrôles du déroulement de la culture, puis alimenter la chambre de culture par un autre milieu nutritif propre à la conservation des cellules cultivées. On peut aussi changer de milieu nutritif en cours de culture, etc.

Ces multiples possibilités de réglage permettent également d'optimiser le fonctionnement du bioréacteur et son rendement, notamment grâce à un réglage précis des quantités utilisées de facteurs de croissance, qui sont des produits très coûteux et dont les concentrations sont un facteur critique très sensible de la culture de cellules en bioréacteur.

On l'a vérifié tout d'abord au moyen d'expériences de maintien de cellules médullaires mononucléées pendant treize jours à une concentration de 10⁵ cellules/ml dans un milieu de culture contenant un mélange de six cytokines différentes (essais A) et dans un milieu de culture identique mais ne contenant pas de cytokines (essais B). Les résultats (qui sont la moyenne de trois expériences) sont indiqués dans le tableau ci-dessous et sont exprimés en termes de fois d'expansion après treize jours d'amplification par rapport au jour 0.

| | Nombre de fois d'expansion | | |
|---|---|---|---|
| | cellules | BFU - e | CFU - GM |
| Essais A | 4,71 ± 1,38 | 1,24 ± 0,75 | 9 ± 4,2 |
| Essais B | 0,12 ± 0,06 | 0,08 ± 0,04 | 0,24 ± 0,12 |
| BFU-e = progéniteurs érythroïdes CFU-GM = progéniteurs granulo-macrophagiques | | | |

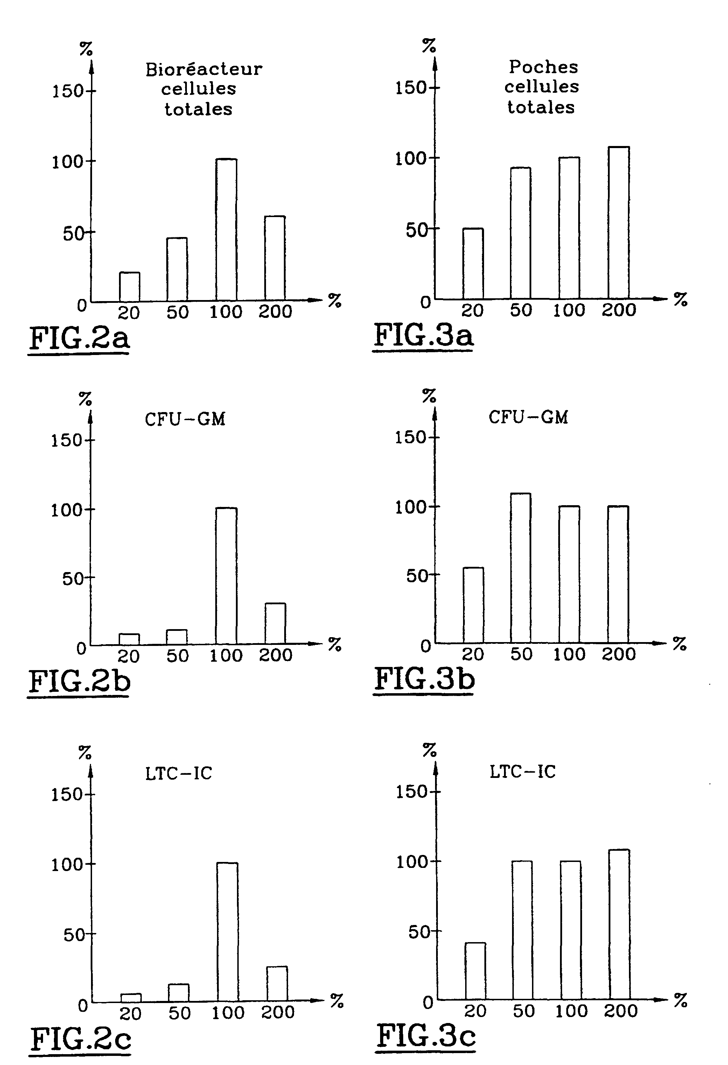

Le rôle de la concentration en facteurs de croissance a été mis en évidence dans des essais comparatifs de culture de cellules en bioréacteur d'une part (c'est-à-dire dans un milieu de culture à perfusion continue ou discontinue de milieu nutritif et de facteurs de croissance) et d'autre part dans des poches (c'est-à-dire en milieu statique), les résultats de ces essais étant représentés par les courbes des figures 2 et 3.

Dans ces figures, on trouve en abscisses la concentration des cytokines en pourcentage d'une concentration optimale correspondant à la valeur 100. En ordonnées, on trouve l'expansion des cellules nucléées totales, des progéniteurs granulo-macrophagiques CFU-GM et des cellules souches LCT-IC, respectivement, en pourcentage de l'expansion obtenue pour la concentration optimale de cytokines correspondant à la valeur 100.

Les cellules nucléées totales comprennent des cellules matures, des progéniteurs et des cellules-souches.

On voit, par comparaison des figures 2a et 3a illustrant l'expansion des cellules totales, que cette expansion est peu sensible à la concentration de cytokines lorsqu'elle varie entre 50 et 200% de la concentration optimale dans un système de culture statique (figure 3a) alors qu'elle est réduite de moitié dans un bioréacteur lorsque la concentration de cytokines est de 50% ou de 200% respectivement de la concentration optimale (figure 2a).

Les mêmes constatations sont faites en ce qui concerne l'expansion des CFU-GM (figures 2b et 3b) et des LTC-IC (figures 2c et 3c).

Les essais dont les résultats sont représentés aux figures 2 et 3 ont été réalisés avec un mélange de six cytokines différentes, pour lequel des expansions maximales de cellules nucléées totales, de CFU-GM et de LTC-IC ont été obtenues dans un bioréacteur avec (à titre indicatif) une concentration de 100 ng de trois cytokines, 5 ng d'une quatrième cytokine et 10 ng d'une cinquième et d'une sixième cytokine. Les figures 2a, 2b et 2c montrent que, quand la concentration de cytokines est inférieure à la valeur optimale, l'expansion des cellules totales, des CFU-GM et des LTC-IC est fonction de la quantité de cytokines et qu'au-delà de la valeur optimale de la concentration, l'expansion diminue nettement.

Dans le mode de réalisation représenté en figure 1, l'enceinte 10 comporte une alimentation "frontale" en milieux nutritifs et en facteurs de croissance. Elle pourrait être équipée, en variante, d'une alimentation par filtration tangentielle comme décrit dans le document EP-A-0474847, cette alimentation comportant une nappe de tubes à parois perméables aux milieux nutritifs.

## Revendications

1. Procédé de culture cellulaire à applications multiples, en particulier pour l'entretien, la prolifération, l'amplification ou la différenciation de cellules dans une enceinte fermée (10), consistant à introduire des cellules dans cette enceinte, à l'alimenter par des débits de gaz de ventilation, de milieu nutritif et de facteurs de croissance, et à récolter les cellules cultivées, **caractérisé en ce qu'**il consiste également :
- à disposer de plusieurs sources (28) de composants ou de milieux nutritifs différents et de plusieurs sources (34) de facteurs de croissance différents,
- à déterminer des compositions et des débits de mélanges des différents composants ou milieux nutritifs et des compositions et des débits de mélanges des différents facteurs de croissance, correspondant à l'une envisagée des applications précitées,
- puis, au cours du déroulement de ladite application, à modifier à intervalles réguliers ou non les compositions et/ou les débits des mélanges de composants ou de milieux nutritifs et des mélanges de facteurs de croissance alimentant l'enceinte (10), ces modifications pouvant être effectuées indépendamment les unes des autres.

2. Procédé selon la revendication 1,
**caractérisé en ce qu'**il consiste à disposer de plusieurs gaz différents, à déterminer une composition et un débit d'un mélange des différents gaz correspondant à ladite application envisagée, à alimenter l'enceinte (10) par ce mélange de gaz et, à intervalles réguliers ou non, à modifier si nécessaire la composition et/ou le débit du mélange de gaz alimentant l'enceinte.

3. Procédé selon la revendication 1 à 2,
**caractérisé en ce qu'**il consiste à contrôler, de façon périodique ou non, le développement des cellules dans ladite enceinte (10) au cours du déroulement de l'application précitée et à effectuer les modifications précitées des compositions et/ou des débits en fonction des résultats de ces contrôles.

4. Procédé selon l'une des revendications précédentes, **caractérisé en ce que** l'alimentation de l'enceinte (10) en milieu nutritif est continue ou discontinue.

5. Procédé selon l'une des revendications précédentes, **caractérisé en ce que** les cellules précitées sont des cellules hématopoïétiques.

6. Dispositif de culture de cellules à applications multiples, en particulier pour l'entretien, la prolifération, l'amplification ou la différenciation de cellules, comprenant une enceinte fermée (10), des membranes microporeuses (14) délimitant une chambre de culture (12) dans ladite enceinte, des moyens (44) d'introduction de cellules dans ladite chambre, des moyens d'alimentation de l'enceinte en gaz de ventilation, en milieu nutritif et en facteurs de croissance, et des moyens (44) de collecte des cellules, **caractérisé en ce qu'**il comprend également des réservoirs (28) de milieux ou de composants nutritifs différents, reliés par des moyens (30) de réglage de débit à un collecteur (32) d'alimentation de ladite enceinte, des réservoirs (34) de facteurs de croissance différents, reliés par des moyens (36) de réglage de débit à un autre ou au même collecteur (38, 32) d'alimentation de l'enceinte, et des moyens (52) de commande des moyens de réglage (30, 36) précités pour déterminer, en fonction d'une application envisagée, la composition et/ou le débit d'un mélange de milieux nutritifs et la composition et/ou le débit d'un mélange de facteurs de croissance, amenés dans l'enceinte par le ou les collecteurs d'alimentation précités, et pour modifier ces compositions et/ou ces débits au cours du déroulement de cette application.

7. Dispositif selon la revendication 6,
**caractérisé en ce que** le ou les collecteurs (32, 38) précités sont reliés à ladite enceinte (10) par une chambre (50) de dégazage et de remise en température.

8. Dispositif selon la revendication 6 ou 7, **caractérisé en ce qu'**il comprend des réservoirs (24) de gaz différents, des moyens (26) de réglage de débit reliant ces réservoirs à un collecteur (18) d'alimentation de ladite enceinte et des moyens (50) de commande des moyens de réglage (26) pour déterminer la composition et le débit d'un mélange de gaz amené dans ladite enceinte en fonction de l'application envisagée et pour modifier si nécessaire cette composition et/ou ce débit au cours du déroulement de l'application.

9. Dispositif selon l'une des revendications 6 à 8, **caractérisé en ce que** les cellules précitées sont des cellules hématopoïétiques.

## Claims

1. A method of growing cells, which method has multiple applications, in particular to the maintenance, proliferation, amplification or differentiation of cells in a closed enclosure (10), and consists of introducing cells into said enclosure, delivering to said enclosure ventilation gases, nutrient media and growth factors, and harvesting the cultivated cells, **characterized in that** it further consists of:
- providing multiple sources (28) of different nutrient media or constituents and multiple sources (34) of different growth factors,
- determining compositions and flow rates of mixtures of the different nutrient media or constituents and compositions and flow rates of mixtures of the different growth factors corresponding to an envisaged application, and then
- during the progress of said application, modifying at regular or irregular intervals the compositions and/or the flow rates of the mixtures of nutrient media or constituents and the mixtures of growth factors delivered to the enclosure (10), which modifications can be effected independently of each other.

2. A method according to claim 1, **characterized in that** it further consists of providing a plurality of different gases, determining a composition and a flow rate of a mixture of the different gases corresponding to said envisaged application, delivering said gas mixture to the enclosure (10) and, if necessary, and at regular or irregular intervals, modifying the composition and/or the flow rate of the gas mixture delivered to the enclosure.

3. A method according to claim 1, **characterized in that** it further consists of periodically or non-periodically testing the development of the cells in said enclosure (10) as the aforementioned application proceeds and modifying said compositions and/or flow rates as a function of the results of said tests.

4. A method according to claim 1, **characterized in that** nutrient medium is delivered to the enclosure (10) continuously or discontinuously.

5. A method according to claim 1, **characterized in that** the aforementioned cells are hematopietic cells.

6. A device for growing cells, which device has multiple applications, in particular to the maintenance, proliferation, amplification or differentiation of cells, and includes a closed enclosure (10), microporous membranes (14) delimiting a growth chamber (12) in said enclosure, means (44) for delivering cells to said chamber, means for delivering ventilation gases, nutrient media and growth factors to the enclosure, and means (44) for harvesting cells, **characterized in that** it further includes containers (28) of different nutrient media or constituents connected by flow rate adjustment means (30) to a delivery manifold (32) of said enclosure, containers (34) of different growth factors connected by flow rate adjustment means (36) to another delivery manifold or the same delivery manifold (38, 32) of the enclosure, and means (52) for controlling the aforementioned adjustment means (30, 36) to determine, as a function of an envisaged application, the composition and/or the flow rate of a mixture of nutrient media and the composition and/or the flow rate of a mixture of growth factors delivered to the enclosure via said delivery manifold or manifolds and to modify said compositions and/or said flow rates as said application proceeds.

7. A device according to claim 6, **characterized in that** said manifold or manifolds (32, 38) are connected to said enclosure (10) by a degassing and temperature adjustment chamber (50).

8. A device according to claim 6, **characterized in that** it includes containers (24) for different gases, flow rate adjustment means (26) connecting said containers to a delivery manifold (18) of said enclosure, and means (50) for controlling the adjustment means (26) to determine the composition and the flow rate of a gas mixture delivered to said enclosure as a function of the envisaged application and to modify said composition and/or said flow rate if necessary as the application proceeds.

9. A device according to claim 6, **characterized in that** said cells are hematopietic cells.

## Patentansprüche

1. Verfahren zum Kultivieren (Züchten) von Zellen, das für viele Anwendungen geeignet ist, insbesondere für die Aufrechterhaltung, Proliferation, Amplifikation oder Differenzierung von Zellen in einem geschlossenen Behälter (10), und darin besteht, dass man in diesen Behälter Zellen einführt, dem Behälter Belüftungsgase, Nährstoffmedium und Wachstumsfaktoren in Form von Strömen zuführt und die kultivierten Zellen erntet,
**dadurch gekennzeichnet, dass** es außerdem darin besteht, dass man:
mehrere Quellen (28) für unterschiedliche Nährstoff-Medien oder - Komponenten und mehrere Quellen (34) für unterschiedliche Wachstumsfaktoren bereitstellt,
die Zusammensetzungen und Strömungsraten der Mischungen von unterschiedlichen Nährstoff-Medien oder -Komponenten und die Zusammensetzungen und Strömungsraten der Mischungen von unterschiedlichen Wachstumsfaktoren, die einer vorgesehenen Anwendung entsprechen, bestimmt und dann während des Ablaufs dieser Anwendung in regelmäßigen oder unregelmäßigen Zeitabständen die Zusammensetzungen und/oder Strömungsraten der Mischungen von Nährstoff-Medien oder -Komponenten und der Mischungen von Wachstumsfaktoren, die dem Behälter (10) zugeführt werden, modifiziert, wobei die Modifikationen unabhängig voneinander durchgeführt werden können.

2. Verfahren nach Anspruch 1, **dadurch gekennzeichnet, dass** es außerdem darin besteht, dass man eine Vielzahl von unterschiedlichen Gasen bereitstellt, die Zusammensetzung und die Strömungsrate einer Mischung der unterschiedlichen Gase, die der vorgesehenen Anwendung entsprechen, bestimmt, diese Gasmischung dem Behälter (10) zuführt und erforderlichenfalls in regelmäßigen oder unregelmäßigen Zeitabständen die Zusammensetzung und/oder die Strömungsrate der dem Behälter zugeführte Gasmischung modifiziert.

3. Verfahren nach Anspruch 1 oder 2, **dadurch gekennzeichnet, dass** es außerdem darin besteht, dass man die Entwicklung der Zellen in dem Behälter (10) periodisch oder nicht-periodisch testet während des Ablaufs der oben genannten Anwendung, und die Zusammensetzungen und/oder Strömungsraten als Funktion der Ergebnisse dieser Tests modifiziert.

4. Verfahren nach einem der vorhergehenden Ansprüche, **dadurch gekennzeichnet, dass** man das Nährstoffmedium kontinuierlich oder diskcontinuierlich dem Behälter (10) zuführt.

5. Verfahren nach einem der vorhergehenden Ansprüche, **dadurch gekennzeichnet, dass** die oben genannten Zellen hämatopoetische Zellen sind.

6. Vorrichtung zum Kultivieren (Züchten) von Zellen, die für mehrere Anwendungen geeignet ist, insbesondere zur Aufrechterhaltung, Proliferation, Amplifikation oder Differenzierung von Zellen, und die umfasst einen geschlossenen Behälter (10), mikroporöse Membranen (14), die eine Kultivierungskammer (12) in dem Behälter begrenzen, Einrichtungen (44) zur Einführung von Zellen in die Kammer, Einrichtungen zur Einführung von Belüftungsgasen, Nährstoff-Medium und Wachstumsfaktoren in den Behälter und Einrichtungen (44) zum Ernten der Zellen, **dadurch gekennzeichnet, dass** sie außerdem umfasst Vorratsbehälter (28) für verschiedene Nährstoff-Medien oder -Komponenten, die mittels Strömungsraten-Einsteilungseinrichtungen (30) mit einer Zuführungssammelleitung (32) des Behälters verbunden sind, Vorratsbehälter (34) für verschiedene Wachstumsfaktoren, die mittels Strömungsraten-Einstellungseinrichtungen (36) mit einer anderen Zuführungssammelleitung oder mit der gleichen Sammelleitung (38, 32) des Behälters verbunden sind, und Einrichtungen (52) zur Kontrolle der oben genannten Einstellungseinrichtungen (30, 36), um die Zusammensetzung und/oder die Strömungsrate einer Mischung von Nährstoffmedien und die Zusammensetzung und/oder Strömungsrate einer Mischung von Wachstumsfaktoren, die dem Behälter mittels der Sammelleitung(en) zugeführt werden, als Funktion des Ablaufs der vorgesehenen Anwendung zu bestimmen und diese Zusammensetzungen und/oder Strömungsraten zu modifizieren, während die Anwendung abläuft.

7. Vorrichtung nach Anspruch 6, **dadurch gekennzeichnet, dass** die Sammelleitung(en) (32, 38) über eine Entgasungs- und Temperatureinstellungskammer (50) mit dem Behälter (10) verbunden ist (sind).

8. Vorrichtung nach Anspruch 6 oder 7, **dadurch gekennzeichnet, dass** sie umfasst Vorratsbehälter (24) für verschiedene Gase, Strömungsraten-Einstellungseinrichtungen (26), welche diese Vorratsbehälter mit einer Zuführungs-Sammelleitung (18) des Gehäuses verbinden, und Einrichtungen (50) zur Kontrolle der Einstellungseinrichtungen (26), um die Zusammensetzung und die Strömungsrate einer dem Behälter zugeführten Gasmischung als Funktion der vorgesehenen Anwendung zu bestimmen und um die Zusammensetzung und/oder Strömungsrate erforderlichenfalls während des Ablaufs der Anwendung zu modifizieren.

9. Vorrichtung nach Anspruch 6, **dadurch gekennzeichnet, dass** die Zellen hämatopoetische Zellen sind.
